(19) **Europäisches Patentamt**

**European Patent Office**

**Office européen des brevets**

(11) Publication number: **0 110 357**
**B1**

(12) **EUROPEAN PATENT SPECIFICATION**

(45) Date of publication of patent specification: **22.04.87**

(21) Application number: **83111859.1**

(22) Date of filing: **26.11.83**

(51) Int. Cl.[4]: **C 07 C 29/15, C 07 C 31/04, C 07 C 31/08, C 07 C 31/10, C 07 C 31/12, C 07 C 31/125, B 01 J 23/78, B 01 J 23/84, B 01 J 23/82**

(54) Process for the production of mixed alcohols.

(30) Priority: **29.11.82 JP 207662/82**
**29.12.82 JP 229834/82**

(43) Date of publication of application:
**13.06.84 Bulletin 84/24**

(45) Publication of the grant of the patent:
**22.04.87 Bulletin 87/17**

(84) Designated Contracting States:
**BE DE FR GB IT NL**

(56) References cited:
**EP-A-0 034 767**
**GB-A-1 205 156**
**GB-A-2 110 558**
**US-A-1 625 626**
**US-A-1 791 568**

(73) Proprietor: **RESEARCH ASSOCIATION FOR PETROLEUM ALTERNATIVES DEVELOPMENT**
**4-2, 1-chome Uchikanda**
**Chiyoda-ku Tokyo (JP)**

(72) Inventor: **Shibata, Masatoshi**
**No. 1720, Kamiizumi Sodegaura-machi**
**Kimitsu-gun Chiba-ken (JP)**
Inventor: **Aoki, Yoshinobu**
**No. 1660, Kamiizumi Sodegaura-machi**
**Kimitsu-gun Chiba-ken (JP)**
Inventor: **Uchiyama, Tsutomu**
**No. 1218-2, Kamiizumi Sodegaura-machi**
**Kimitsu-gun Chiba-ken (JP)**
Inventor: **Uchiyama, Soichi**
**No. 1660, Kamiizumi Sodegaura-machi**
**Kimitsu-gun Chiba-Ken (JP)**

(74) Representative: **Türk, Dietmar, Dr. rer. nat. et al**
**Türk, Gille + Hrabal Patentanwälte Bruckner Strasse 20**
**D-4000 Düsseldorf 13 (DE)**

## Description

### Background of the invention

In view of a rise in price of gasoline for cars due to the aggravation of oil situation, an attempt to produce inexpensive car fuel by adding mixed alcohols to gasoline have been made in recent years. The reason why mixed alcohols are used as an alcohol component to be added to gasoline is that if methanol alone is added to gasoline, it combines together with water in gasoline to form a water/methanol mixture, resulting in the formation of two layers, i.e., a gasoline layer and a water/methanol mixed layer, in a storage tank.

Various methods of producing such mixed alcohols have been proposed. Japanese Patent Application Laid-Open No. 7727/1981, for example, discloses a process for producing mixed alcohols from synthesis gas by the use of a rhodium-base catalyst. This process, however, is not preferred in that large amounts of by-products such as acetic acid and aldehyde result. In addition, as catalysts for use in the production of mixed alcohols from synthesis gas, a ruthenium-base catalyst (Japanese Patent Application Laid-Open No. 82327/1982), alkali metal-modified ones of a zinc-chromium catalyst and a copper-zinc catalyst (Japanese Patent Application Laid-Open No. 10689/1982), and a copper-cobalt catalyst (Japanese Patent Application Laid-Open No. 85530/1980) are known. Methods utilizing these catalysts, however, should be performed under elevated pressures. This will need expensive equipment and cause many side reactions. Hence they cannot be said to be advantageous for practical use.

### Summary of the invention

The present invention is intended to overcome the above-described problems of conventional methods, and an object of the invention is to provide a process for producing mixed alcohols from synthesis gas with efficiency under relatively low pressures.

One embodiment of the present invention relates to a process for producing a mixed alcohol comprising methanol and higher alcohols than methanol by contacting synthesis gas with a catalyst, wherein the catalyst is a solid substance prepared by:

calcining a mixture of $(A_1)$ a copper compound, $(B_1)$ a nickel compound, and (C) a compound of at least one metal selected from the metals belonging to Groups II, III and IV and the fourth period of Groups V, VI and VII of the Periodic Table;

impregnating the above-calcined product with (D) an alkali metal compound and/or an alkaline earth metal compound;

calining the resulting mixture; and

reducing the thus-calcined product.

The other embodiment of the present invention relates to a process for producing a mixed alcohol comprising methanol and higher alcohols than methanol by contacting synthesis gas with a catalyst, wherein the catalyst is a solid substance prepared by:

calcining a mixture of $(A_2)$ a zinc compound, $(B_2)$ a compound of at least one metal selected from iron, cobalt, and nickel, and (C) a compound of at least one metal selected from the metals belonging to Groups II, III and IV and the fourth period of Groups V, VI and VII of the Periodic Table;

impregnating the above-calcined product with (D) an alkali metal compound and/or an alkaline earth metal compound;

calcining the resulting mixture; and

reducing the thus-calcined product.

### Detailed description of the invention

A method of preparing the catalyst of the invention will hereinafter be explained in detail. In the preparation of the catalyst, Compounds (A), (B) and (C) are first mixed and calcined.

As Compound (A), Compound $(A_1)$ or Compound $(A_2)$ can be used.

As Compound $(A_1)$ used herein, any suitable compound containing copper can be listed. Usually water-soluble compounds are preferred. Suitable examples of copper compounds include copper nitrate, copper sulfate, and copper chloride.

As Compound $(A_2)$ used herein, any suitable compound containing zinc can be listed. Usually water-soluble compounds are preferred. Suitable examples of zinc compounds include zinc nitrate, zinc sulfate, and zinc chloride.

As Compound (B), Compound $(B_1)$ or Compound $(B_2)$ can be used.

Compound $(B_1)$ used herein, any suitable compound containing nickel can be listed. Particularly preferred are water-soluble compounds. Suitable examples of nickel compounds include nickel nitrate, nickel sulfate, and nickel chloride.

Compound $(B_2)$ is a compound containing at least one of iron, cobalt, and nickel. Particularly preferred are water-soluble compounds. Suitable examples are the nitrates, sulfates, and chlorides of the metals.

Compound $(A_1)$ can be used in combination with Compound $(B_1)$. Similar by, Compound $(A_2)$ can be used in combination with Compound $(B_2)$.

Compound (C) is a compound of at least one metal selected from the metals belonging to Groups II, III and IV, and the fourth period of Groups V, VI and VII of the Periodic Table. Typical examples of the metals belonging to Groups II, III and IV of the Periodic Table are magnesium, calcium, zinc, boron, aluminum, gallium, lanthanum, silicon, germanium, titanium, tin, and zirconium. Suitable examples of the metals belonging to the fourth period of Groups V, VI and VII of the Period Table are vanadium, chromium, and manganese. As Compound (C), various compounds of the metals as described above, such as the nitrates, sulfates, chlorides, and oxides thereof, can be used. Particularly preferred are water-soluble compounds.

As Compound (C), salt of titanium is one of the preferable compounds. Especially sulfate of it is preferable. Other salts such as titanium tetrachloride are undesirable here. When dissolved into water, titanium tetrachloride, for instance, is difficult to treat since it fumes and is hydrolyzed not to be dispersed homogeneously. Moreover, other salts are insoluble in water.

Titanium sulfate is a favourable salt for dispersing of titanium into catalyst as is described above, but sulfate radicals tend to remain in catalyst. When sulfur portion is 0.5% by weight or more, catalytic activity is scarcely observed.

Accordingly, when titanium sulfate is employed for producing catalyst, it is inevitable to remove sulfate radicals to make sulfur portion less than 0.5% by weight after precipitate results.

After earnest researches in removal of sulfate radicals, we have found that following two processes are desirable.

One of the processes is to repeat washing with aqueous solution of sodium chloride after the precipitate results, and to exchange sulfate radicals with chlorine ions to reduce sulfate radicals, and thus make sulfur portion less than 0.5% by weight. Thereupon, the concentration of the aqueous solution of sodium chloride is desired to be 0.1 mole per liter—5 mole per liter.

Another process is to adjust pH at co-precipitating by addition of sodium carbonate. There, once co-precipitation is made at pH 9.0 or more, after that the washing with plain water can reduce the sulfate radicals to make the sulfur portion less than 0.5% by weight. When pH is less than 9.0, activity does not arise sufficiently.

In the preparation of the catalyst of the invention, Compound (A), (B) and (C) are first mixed and calcined.

Compounds (A), (B) and (C) can be mixed by techniques such as a co-precipitation method, a kneading method, and a dipping method. In accordance with the co-precipitation method, for example, they are added to water to form aqueous solutions or suspensions, which are then mixed and co-precipitated by adjusting the pH through addition of a co-precipitating agent such as sodium carbonate, sodium hydroxide, and potassium hydroxide at room temperature or at elevated temperatures. Then, the resulting precipitate is aged, if necessary, and washed with water, dried and calcined at a temperature of from 200 to 500°C.

The above-calcined product is then impregnated with Compound (D), i.e., an alkali metal compound and/or an alkaline earth metal compound. Compound (D) is preferably water-soluble. Suitable examples include sodium carbonate and magnesium acetate. In the impregnation of the calcined product, Compound (D) is used as an aqueous solution; that is, the calcined product is impregnated with an aqueous solution of Compound (D). After the process of impregnation, the resulting mixture should be calcined again. This calcination is usually performed at a temperature of from 100 to 500°C.

Although the composition of the thus-calcined product varies with the amounts of Compounds (A), (B), (C) and (D) being added, it is necessary for the molar ratio of (A) to (B) to (C) to (D) (calculated as oxide) to be controlled so that $0.05<(A)<0.7$, $0.01<(B)<0.7$, $0.01<(C)<0.7$, and $0.005<(D)<0.3$.

The calcined product is then reduced. This reduction is sufficient to be performed at a temperature of from 200 to 400°C by the use of a reducing atmosphere, for example, in the presence of hydrogen or carbon monoxide.

The thus-prepared solid substance, is used as the catalyst of the invention.

Although compounds (A), (B), (C) and (D) can be mixed and calcined simultaneously, Compound (D) of alkali or alkaline earth metal compound is dispersed only insufficiently and unevenly in the final product by such a procedure. Hence this procedure fails to produce the desired catalyst.

In the process of the invention, the solid substance as prepared above is used as a catalyst, and synthesis gas, i.e., a mixed gas of hydrogen and carbon monoxide, is contacted with the catalyst to produce a mixed alcohol. The composition of the synthesis gas to be used as a feed in the process of the invention is not critical. In general, however, it is preferred to use synthesis gas in which the molar ratio of hydrogen to carbon monoxide is within the range of from 1:3 to 3:1.

Other reaction conditions for the process of the invention are not critical and can be determined appropriately. The reaction temperature is usually from 200 to 550°C and preferably from 240 to 450°C; the reaction pressure may be relatively low, in general, ranges between 20 and 200 kilograms per square centimeter (by gauge) and preferably between 40 and 100 kilograms per square centimeter (by gauge); and the gas hourly space velocity (GHSV) is from 500 to 100,000 per hour and preferably from 1,000 to 50,000 per hour.

The process of the invention as described above produces mixed alcohols comprising methanol and higher alcohols than methanol, such as ethanol, propanol, and butanol, and other compounds such as aldehydes and esters. The selectivity of the mixed alcohol is high in the process of the invention. This is one of the advantages of the present invention. Another advantage is that the costs of equipment and operation, for example, can be greatly reduced, since the reaction pressure in the process of the invention is sufficient to be relatively low. Furthermore the proportion of alcohols other than methanol in the mixed alcohol as produced by the process of the invention is relatively high, and thus the mixed alcohol is suitable for use as an alcohol component to be compounded to gasoline.

The present invention is described in greater detail with reference to the following examples.

Example 1

An aqueous solution (Aqueous Solution I) (2.5 liters) containing 48.3 grams of copper nitrate (3 hydrate), 29.1 grams of nickel nitrate (6 hydrate), and 59.5 grams of zinc nitrate (6 hydrate) was prepared and heated to 60°C. Separately 2.5 liters of an aqueuous solution (Aqueous Solution II) containing 81.3 grams of sodium carbonate (anhydrous) was prepared and heated to 60°C.

These aqueous solutions were mixed rapidly and, after completion of precipitation, aged. Then the resulting mixture was filtered, and the precipitate thus obtained was washed sufficiently with water, dried at 120°C for about 12 hours and then calcined at 450°C for 2 hours.

The thus-calcined product was impregnated with an aqueous solution (Aqueous Solution III) containing 6.8 grams of sodium carbonate (anhydrous) and dried at 120°C for about 12 hours. Then graphite was added, and the resulting mixture was pelletized and pulverized to produce 16—32 mesh grains. The thus-prepared catalyst precursor had a composition of

Cu:Ni:Zn:Na=0.36:0.18:0.36:0.10

(molar ratio).

Then 1 milliliter of the catalyst precursor was packed in a reaction tube of stainless steel. While passing a 1:9 (molar ratio) mixture of carbon monoxide and nitrogen as a reducing gas through the reaction tube at a gas hourly space velocity (GHSV) of 4,000 per hour, the catalyst precursor was gradually heated and reduced at 240°C for 5—20 hours to produce a catalyst.

A synthesis gas (carbon monoxide:hydrogen=1:2 (molar ratio)) was introduced into the reaction tube at a gas hourly space velocity (GHSV) of 4,000 per hour. The pressure was gradually increased to 50 kilograms per square centimeter (by gauge). Then the temperature was increased to a reaction temperature at which the conversion of carbon monoxide (excluding the one converted into carbon dioxide) reached about 20%. The reaction products were passed through a tube maintained at 200°C, without being condensed at the outlet of the reaction tube, and introduced into a gas chromatography instrument where they were analyzed. The column filler as used in this gas chromatography analysis was a mixture of activated carbon, Porapak-Q (produced by Water Co.) and Porapak-R (produced by Water Co.). The results are shown in Table 1.

Example 2

A catalyst precursor was prepared in the same manner as in Example 1 except that 2.5 liters of an aqueous solution containing 48.3 grams of copper nitrate (3 hydrate), 29.1 grams of nickel nitrate (6 hydrate), and 75.0 grams of aluminum nitrate (9 hydrate) was used as Aqueous Solution I and 2.5 liters of an aqueous solution containing 90.2 grams of sodium carbonate (anhydrous) as Aqueous Solution II. This catalyst precursor had a composition of

Cu:Ni:Al:Na=0.36:0.18:0.36:0.10

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

Example 3

A catalyst precursor was prepared in the same manner as in Example 1 except that 2.5 liters of an aqueous solution containing 48.3 grams of copper nitrate (3 hydrate), 29.1 grams of nickel nitrate (6 hydrate), and 75.0 grams of aluminum nitrate (9 hydrate) was used as Aqueous Solution I, 2.5 liters of an aqueous solution containing 90.2 grams of sodium carbonate (anhydrous) as Aqueous Solution II, and an aqueous solution containing 13.7 grams of magnesium acetate (4 hydrate) as Aqueous Solution III for the process of impregnation. This catalyst precursor had a composition of

Cu:Ni:Al:Mg=0.36:0.18:0.36:0.10

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

Example 4

A catalyst precursor was prepared in the same manner as in Example 1 except that 2.5 liters of an aqueous solution containing 48.3 grams of copper nitrate (3 hydrate), 29.1 grams of nickel nitrate (6 hydrate), and 79.9 grams of gallium nitrate (8 hydrate) was used as Aqueous Solution I, and 2.5 liters of an aqueous solution containing 91.6 grams of sodium carbonate (anhydrous) as Aqueous Solution II. This catalyst precursor had a composition of

$$Cu:Ni:Ga:Na=0.36:0.18:0.36:0.10$$

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

Example 5

A catalyst precursor was prepared in the same manner as in Example 1 except that 2.5 liters of an aqueous solution containing 48.3 grams of copper nitrate (3 hydrate) and 29.1 grams of nickel nitrate (6 hydrate) was used as Aqueous Solution I, and 2.5 liters of an aqueous solution containing 61.7 grams of water glass ($SiO_2$ content: 28.6% by weight) and 37.2 grams of sodium carbonate (anhydrous) as Aqueous Solution II. This catalyst precursor had a composition of

$$Cu:Ni:Si:Na=0.36:0.18:0.36:0.10$$

(molar ratio).

The catalyst precursor was reduced in the same manner as in Example 1 to form a catalyst. Using the thus-prepared catalyst, the production mixed alcohol from synthesis gas was performed in the same manner as in Example 1. The results are shown in Table 1.

Example 6

A catalyst precursor was prepared in the same manner as in Example 1 except that 2.5 liters of an aqueous solution containing 48.3 grams of copper nitrate (3 hydrate), 29.1 grams of nickel nitrate (6 hydrate), and 64.4 grams of zirconium oxychloride (8 hydrate) was used as Aqueous Solution I, and 2.5 liters of an aqueous solution containing 63.7 grams of sodium carbonate (anhydrous) as Aqueous Solution II. This catalyst precursor had a composition of

$$Cu:Ni:Zr:Na=0.36:0.18:0.36:0.10$$

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

Example 7

An aqueous solution (2.5 liters) containing 48.3 grams of copper nitrate (3 hydrate), 29.1 grams of nickel nitrate (6 hydrate), and 161.1 grams of titanium sulfate ($Ti(SO_4)_2$ content: 29.8% by weight) was prepared and heated to 60°C. Separately 2.5 liters of an aqueous solution containing 128.0 grams of sodium carbonate (anhydrous) was prepared and heated to 60°C. These aqueous solutions were mixed rapidly and, after completion of precipitation, aged. The resulting mixture was filtered, and the precipitate thus obtained was treated with an aqueous solution of sodium chloride (concentration: 0.5 mole per liter) and further washed sufficiently with water.

Thereafter the same procedure as in Example 1 was performed to form a catalyst precursor. This catalyst precursor had a composition of

$$Cu:Ni:Ti:Na=0.36:0.18:0.36:0.10$$

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

Example 8

A catalyst precursor was prepared in the same manner as in Example 1 except that 2.5 liters of an aqueous solution containing 48.3 grams of copper nitrate (3 hydrate), 29.1 grams of nickel nitrate (6 hydrate), and 80.0 grams of chromium nitrate was used as Aqueous Solution I, and 2.5 liters of an aqueous solution containing 90.8 grams of sodium carbonate (anhydrous) as Aqueous Solution II. This catalyst precursor had a composition of

$$Cu:Ni:Cr:Na=0.38:0.19:0.31:0.12$$

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

Example 9

A catalyst precursor was prepared in the same manner as in Example 1 except that 2.5 liters of an aqueous solution containing 48.3 grams of copper nitrate (3 hydrate), 29.1 grams of nickel nitrate (6 hydrate), and 86.6 grams of lanthanum nitrate (6 hydrate) was used as Aqueous Solution I, and 2.5 liters of an aqueous solution containing 74.2 grams of sodium carbonate (anhydrous) as Aqueous Solution II. This catalyst precursor had a composition of

$$Cu:Ni:La:Na=0.36:0.18:0.36:0.10$$

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

Example 10

A catalyst precursor was prepared in the same manner as in Example 1 except that 2.5 liters of an aqueous solution containing 48.3 grams of copper nitrate (3 hydrate), 29.1 grams of nickel nitrate (6 hydrate), and 86.6 grams of lanthanum nitrate (6 hydrate) was used as Aqueous Solution I, 2.5 liters of an aqueous solution containing 74.2 grams of sodium carbonate (anhydrous) as Aqueous Solution II, and an aqueous solution containing 13.7 grams of magnesium acetate (4 hydrate) as Aqueous Solution III for the process of impregnation. This catalyst precursor had a composition of

$$Cu:Ni:La:Mg=0.36:0.18:0.36:0.10$$

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

Example 11

A catalyst precursor was prepared in the same manner as in Example 1 except that 2.5 liters of an aqueous solution containing 48.3 grams of copper nitrate (3 hydrate), 29.1 grams of nickel nitrate (6 hydrate), and 25.6 grams of magnesium nitrate (6 hydrate) was used as Aqueous Solution I, and 2.5 liters of an aqueous solution containing 50.3 grams of sodium carbonate (anhydrous) as Aqueous Solution II. This catalyst precursor had a composition of

$$Cu:Ni:Mg:Na=0.43:0.22:0.22:0.13$$

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 1.

TABLE 1

| Example | Reaction temperature (°C) | Conversion of carbon monoxide[1] (%) | Selectivity of alcohol[2] (%) | Composition of mixed alcohol (% by weight) | | | |
|---|---|---|---|---|---|---|---|
| | | | | Methanol | Ethanol | Propanol | Butanol and higher alcohols than butanol |
| 1 | 311 | 20 | 70 | 88 | 8 | 3 | 1 |
| 2 | 305 | 22 | 55 | 55 | 29 | 8 | 8 |
| 3 | 315 | 21 | 68 | 89 | 8 | 2 | 1 |
| 4 | 307 | 19 | 53 | 58 | 27 | 8 | 7 |
| 5. | 312 | 21 | 52 | 53 | 28 | 11 | 8 |
| 6 | 333 | 21 | 43 | 82 | 10 | 4 | 4 |
| 7 | 307 | 19 | 46 | 45 | 37 | 10 | 8 |
| 8 | 335 | 20 | 69 | 87 | 9 | 3 | 1 |
| 9 | 331 | 20 | 55 | 77 | 15 | 1 | 1 |
| 10 | 334 | 19 | 52 | 78 | 14 | 6 | 2 |
| 11 | 319 | 21 | 47 | 57 | 30 | 8 | 5 |

Note:

[1] Conversion of Carbon Monoxide $= \dfrac{\text{Amount of Carbon Monoxide Converted into Alcohol (moles)} - \text{Amount of Carbon Dioxide Formed (moles)}}{\text{Amount of Carbon Monoxide Introduced (moles)}} \times 100$

[2] Selectivity of Alcohol $= \dfrac{\text{Amount of Carbon Monoxide Converted into Alcohol (moles)}}{\text{Amount of Carbon Monoxide Converted (moles)} - \text{Amount of Carbon Dioxide Formed (moles)}} \times 100$

0 110 357

Example 12

An aqueous solution (Aqueous Solution I) (1.5 liters) containing 24.2 grams of copper nitrate (3 hydrate), 29.1 grams of nickel nitrate (6 hydrate), and 80 grams of titanium sulfate ($Ti(SO_4)_2$ content: 29.8% by weight) was prepared and heated to 60°C. Separately 1.5 liters of an aqueous solution (Aqueous Solution II) containing 66.3 grams of sodium carbonate (anhydrous) was prepared and heated to 60°C. These aqueous solutions was mixed rapidly and, after completion of precipitation, aged. The resulting mixture was filtered, and the thus-obtained precipitate was treated with an aqueous sodium chloride solution (concentration: 0.5 mole per liter) and washed sufficiently with water.

The precipitate was dried at 120°C for about 12 hours and then calcined at 450°C for 2 hours.

The thus-calcined product was impregnated with 38 milliliters of an aqueous solution (Aqueous Solution III) of sodium carbonate (concentration: 1.0 mole per liter), and dried at 120°C for about 12 hours. Then graphite was added, and the resulting mixture was pelletized and pulverized to form 16—32 mesh grains. The thus-prepared catalyst precursor had a composition of

Cu:Ni:Ti:Na=1:1:1:0.38

(molar ratio).

The catalyst precursor was reduced in the same manner as in Example 1 to form a catalyst. Using the thus-prepared catalyst, the production of mixed alcohol from synthesis gas was performed in the same manner as in Example 1. The results are shown in Table 2.

Example 13

A catalyst percursor was prepared in the same manner as in Example 12 except that 1.5 liters of an aqueous solution containing 16.1 grams of copper nitrate (3 hydrate), 29.1 grams of nickel nitrate (6 hydrate), and 106.7 grams of titanium sulfate (the same as used in Example 12) was used as Aqueous Solution I, and 1.5 liters of an aqueous solution containing 79.5 grams of sodium carbonate (anhydrous) as Aqueous Solution II. This catalyst precursor had a composition of

Cu:Ni:Ti:Na=2:3:4:1.15

(molar ratio).

The catalyst precursor was reduced in the same manner as in Example 12 to form a catalyst. Using the thus-prepared catalyst, the production of mixed alcohol from synthesis gas was performed in the same manner as in Example 12. The results are shown in Table 2.

Example 14

A catalyst precursor was prepared in the same manner as in Example 1 except that 1.5 liters of an aqueous solution containing 24.1 grams of copper nitrate (3 hydrate), 29.1 grams of nickel nitrate (6 hydrate), and 28.7 grams of manganese nitrate (6 hydrate) was used as Aqueous Solution I, 1.5 liters of an aqueous solution containing 39.8 grams of sodium carbonate (anhydrous) as Aqueous Solution II, and 38 milliliters of a solution of sodium carbonate (concentration: 1.0 mole per liter) as Aqueous Solution III. This catalyst precursor had a composition of

Cu:Ni:Mn:Na=1:1:1:0.38

(molar ratio).

The catalyst precursor was reduced in the same manner as in Example 12 to form a catalyst. Using the thus-prepared catalyst, the production of mixed alcohol from synthesis gas was performed in the same manner as in Example 12. The results are shown in Table 2.

Example 15

A catalyst precursor was prepared in the same manner as in Example 1 except that 1.5 liters of an aqueous solution containing 14.5 grams of copper nitrate (3 hydrate), 8.7 grams of nickel nitrate (6 hydrate), and 60.3 grams of manganese nitrate (6 hydrate) was used as Aqueous Solution I, 1.5 liters of an aqueous solution containing 39.8 grams of sodium carbonate (anhydrous) as Aqueous Solution II, and 38 milliliters of a solution of sodium carbonate (concentration: 1.0 mole per liter) as Aqueous Solution III. This catalyst precursor had a composition of

Cu:Ni:Mn:Na=2:1:7:1.28

(molar ratio).

The catalyst precursor was reduced in the same manner as in Example 12 to form a catalyst. Using the thus-prepared catalyst, the production of mixed alcohol from synthesis gas was performed in the same manner as in Example 12. The results are shown in Table 2.

Example 16

A catalyst precursor was prepared in the same manner as in Example 1 except that 1.5 liters of an aqueous solution containing 50.7 grams of copper nitrate (3 hydrate), 8.7 grams of nickel nitrate (6 hydrate), and 17.2 grams of manganese nitrate (6 hydrate) was used as Aqueous Solution I, 1.5 liters of an aqueous solution containing 39.8 grams of sodium carbonate (anhydrous) as Aqueous Solution II, and 38 milliliters of a solution of sodium carbonate (concentration: 1.0 mole per liter) as Aqueous Solution III. This catalyst precursor had a composition of

$$Cu:Ni:Mn:Na = 7:1:2:1.28$$

(molar ratio).

The catalyst precursor was reduced in the same manner as in Example 12 to form a catalyst. Using the thus-prepared catalyst, the production of mixed alcohol from synthesis gas was performed in the same manner as in Example 12. The results are shown in Table 2.

## TABLE 2

| Example | Reaction temperature (°C) | Conversion of carbon monoxide[1] (%) | Selectivity of alcohol[2] (%) | Composition of mixed alcohol (% by weight) | | | |
|---|---|---|---|---|---|---|---|
| | | | | Methanol | Ethanol | Propanol | Butanol and higher alcohols than butanol |
| 12 | 318 | 21 | 51 | 31 | 41 | 13 | 15 |
| 13 | 308 | 20 | 50 | 33 | 39 | 12 | 16 |
| 14 | 251 | 19 | 62 | 43 | 49 | 6 | 2 |
| 15 | 284 | 20 | 80 | 56 | 33 | 1 | 10 |
| 16 | 256 | 20 | 64 | 45 | 48 | 2 | 5 |

Note:
[1], [2] Same as in Table 1.

Example 17

An aqueous solution (2.5 liters) containing 59.5 grams of zinc nitrate (6 hydrate), 29.1 grams of cobalt nitrate (6 hydrate), and 25.6 grams of magnesium nitrate was prepared and heated to 60°C. Separately 2.5 liters of an aqueous solution containing 88.3 grams of sodium carbonate (anhydrous) as a co-precipitating agent was prepared and heated to 60°C. These aqueous solutions were mixed rapidly and, after completion of precipitation, aged. Then the resulting mixture was filtered, and the precipitate thus obtained was washed sufficiently with water.

The thus-obtained co-precipitate was dried at 120°C for about 12 hours and then calcined at 450°C for 2 hours.

The thus-calcined product was impregnated with an aqueous solution containing 3.4 grams of sodium carbonate (anhydrous) and then dried at 120°C for about 12 hours. Then graphite was added, and the resulting mixture was pelletized and pulverized to form 16—32 mesh grains. This catalyst precursor had a composition of

$$Zn:Co:Mg:Na=0.43:0.22:0.22:0.13$$

(molar ratio).

Then 1 milliliter of the catalyst precursor was packed in a reaction tube of stainless steel. While passing a 1:9 (molar ratio) mixture of carbon monoxide and nitrogen as a reducing gas through the reaction tube at a gas hourly space velocity (GHSV) of 4,000 per hour, the catalyst precursor was gradually heated and reduced at 240°C for 12 hours to produce a catalyst.

A synthesis gas (carbon monoxide:hydrogen=1:2 (molar ratio)) was introduced into the reaction tube at a gas hourly space velocity (GHSV) of 4,000 per hour. The pressure was increased to 50 kilograms per square centimeter (by gauge). Then the temperature was increased to a reaction temperature at which the conversion of carbon monoxide (excluding the one converted into carbon dioxide) reached about 20%.

The reaction products were introduced, without being condensed at the outlet of the reaction tube, through a tube maintained at 200°C into a gas chromatography instrument where they were analyzed. The column filler as used in this gas chromatography analysis was a mixture of activated carbon, Porapak-Q (produced by Water Co.), and Porapak-N (produced by Water Co.). The results are shown in Table 3.

Example 18

The procedure of Example 17 was repeated wherein the preparation of the catalyst was performed using the following compounds:

| | |
|---|---|
| zinc nitrate (6 hydrate) | 59.5 grams |
| cobalt nitrate (6 hydrate) | 29.1 grams |
| aluminum nitrate (9 hydrate) | 75.0 grams |
| sodium carbonate (anhydrous) (co-precipitating agent) | 90.2 grams |
| sodium carbonate (anhydrous) (for impregnation) | 3.4 grams |

Composition of catalyst precursor:

$$Zn:Co:Al:Na=0.36:0.18:0.36:0.10 \text{ (molar ratio)}$$

The results are shown in Table 3.

Example 19

The procedure of Example 17 was repeated wherein the preparation of the catalyst was performed using the following compounds:

| | |
|---|---|
| zinc nitrate (6 hydrate) | 59.5 grams |
| cobalt nitrate (6 hydrate) | 29.1 grams |
| aluminum nitrate (9 hydrate) | 75.0 grams |
| sodium carbonate (anhydrous) (for co-precipitation) | 90.2 grams |
| magnesium acetate (4 hydrate) | 13.7 grams |

Composition of catalyst precursor:

Zn:Co:Al:Mg=0.36:0.18:0.36:0.10 (molar ratio)

The results are shown in Table 3.

Example 20

The procedure of Example 17 was repeated wherein the preparation of the catalyst was performed using the following compounds:

| | |
|---|---|
| zinc nitrate (6 hydrate) | 59.5 grams |
| cobalt nitrate (6 hydrate) | 29.1 grams |
| gallium nitrate (8 hydrate) | 79.9 grams |
| sodium carbonate (anhydrous) (for co-precipitation) | 89.2 grams |
| sodium carbonate (anhydrous) (for impregnation) | 3.4 grams |

Composition of catalyst precursor:

Zn:Co:Ga:Na=0.36:0.18:0.36:0.10 (molar ratio)

The results are shown in Table 3.

Example 21

The procedure of Example 17 was repeated wherein the preparation of the catalyst was performed using the following compounds:

| | |
|---|---|
| zinc nitrate (6 hydrate) | 59.5 grams |
| cobalt nitrate (6 hydrate) | 29.1 grams |
| water glass ($SiO_2$ content: 28.6% by weight) | 61.7 grams |
| sodium carbonate (anhydrous) (for co-precipitation) | 35.3 grams |
| sodium carbonate (anhydrous) (for impregnation) | 3.4 grams) |

Composition of catalyst precursor:

Zn:Co:Si:Na=0.36:0.18:0.36:0.10 (molar ratio)

The results are shown in Table 3.

Example 22

The procedure of Example 17 was repeated wherein the preparation of the catalyst was performed using the following compounds:

| | |
|---|---|
| zinc nitrate (6 hydrate) | 59.5 grams |
| cobalt nitrate (6 hydrate) | 29.1 grams |
| zirconium oxychloride (8 hydrate) | 64.4 grams |
| sodium carbonate (anhydrous) (for co-precipitation) | 63.8 grams |
| potassium carbonate (anhydrous) (for impregnation) | 4.2 grams |

Composition of catalyst precursor:

Zn:Co:Zr:K=0.36:0.18:0.36:0.10 (molar ratio)

The results are shown in Table 3.

Example 23

The procedure of Example 17 was repeated wherein the preparation of the catalyst was performed using the following compounds:

| | |
|---|---|
| zinc nitrate (6 hydrate) | 59.5 grams |
| cobalt nitrate (6 hydrate) | 29.1 grams |
| chromium nitrate (9 hydrate) | 80.0 grams |
| sodium carbonate (anhydrous) (for co-precipitation) | 92.5 grams |
| sodium carbonate (anhydrous) (for impregnation) | 3.4 grams |

Composition of catalyst precursor:

Zn:Co:Cr:Na=0.36:0.18:0.36:0.10 (molar ratio)

The results are shown in Table 3.

Example 24

The procedure of Example 17 was repeated wherein the preparation of the catalyst was performed using the following compounds:

| | |
|---|---|
| zinc nitrate (6 hydrate) | 59.5 grams |
| cobalt nitrate (6 hydrate) | 29.1 grams |
| lanthanum nitrate (6 hydrate) | 86.6 grams |
| sodium carbonate (anhydrous) (for co-precipitation) | 74.2 grams |
| magnesium acetate (4 hydrate) | 13.7 grams |

Composition of catalyst precursor:

Zn:Co:La:Mg=0.36:0.18:0.36:0.10 (molar ratio)

The results are shown in Table 3.

13

Example 25

The procedure of Example 17 was repeated wherein the preparation of the catalyst was performed using the following compounds:

| | |
|---|---|
| zinc nitrate (6 hydrate) | 59.5 grams |
| nickel nitrate (6 hydrate) | 29.1 grams |
| aluminum nitrate (9 hydrate) | 75.0 grams |
| sodium carbonate (anhydrous) (for co-precipitation) | 81.3 grams |
| potassium carbonate (anhydrous) (for impregnation) | 4.2 grams |

Composition of catalyst precursor:

$$Zn:Ni:Al:Na=0.36:0.18:0.36:0.10 \text{ (molar ratio)}$$

The results are shown in Table 3.

Example 26

The procedure of Example 17 was repeated wherein the preparation of the catalyst was performed using the following compounds:

| | |
|---|---|
| zinc nitrate (6 hydrate) | 59.5 grams |
| nickel nitrate (6 hydrate) | 29.1 grams |
| zirconium oxychloride (8 hydrate) | 64.4 grams |
| sodium carbonate (anhydrous) (for co-precipitation) | 70.7 grams |
| potassium carbonate (anhydrous) (for impregnation) | 4.2 grams |

Composition of catalyst precursor:

$$Zn:Ni:Zr:Na=0.36:0.18:0.36:0.10 \text{ (molar ratio)}$$

The results are shown in Table 3.

Example 27

The procedure of Example 17 was repeated wherein the preparation of the catalyst was performed using the following compounds:

| | |
|---|---|
| zinc nitrate (6 hydrate) | 59.5 grams |
| nickel nitrate (6 hydrate) | 29.1 grams |
| Aqueous titanium sulfate solution ($Ti(SO_4)_2$ content: 29.8% by weight) | 161.1 grams |
| sodium carbonate (anhydrous) (for co-precipitation) | 133.0 grams |

(After co-precipitation followed by filtration, the resulting co-precipitate was washed with water and further with an aqueous solution of NaCl (0.5 mole per liter) to remove $SO_4^{2-}$).

| | |
|---|---|
| sodium carbonate (anhydrous) (for impregnation) | 4.2 grams |

Composition of catalyst precursor:

Zn:Ni:Ti:Na=0.34:0.17:0.34:0.15 (molar ratio)

The results are shown in Table 3.

Example 28
The procedure of Example 17 was repeated wherein the preparation of the catalyst was performed using the following compounds:

| | |
|---|---|
| zinc nitrate (6 hydrate) | 59.5 grams |
| iron nitrate (9 hydrate) | 40.4 grams |
| aluminum nitrate (9 hydrate) | 75.0 grams |
| sodium carbonate (anhydrous) (for co-precipitation) | 93.4 grams |
| sodium carbonate (anhydrous) (for impregnation) | 3.4 grams |

Composition of catalyst precursor:

Zn:Fe:Al:Na=0.36:0.18:0.36:0.10 (molar ratio)

The results are shown in Table 3.

Example 29
The procedure of Example 17 was repeated wherein the preparation of the catalyst was performed using the following compounds:

| | |
|---|---|
| zinc nitrate (6 hydrate) | 59.5 grams |
| iron nitrate (9 hydrate) | 40.4 grams |
| zirconium oxychloride (8 hydrate) | 64.4 grams |
| sodium carbonate (anhydrous) (for co-precipitation) | 89.3 grams |
| sodium carbonate (anhydrous) (for impregnation) | 3.4 grams |

Composition of catalyst precursor:

Zn:Fe:Zr:Na=0.36:0.18:0.36:0.10 (molar ratio)

The results are shown in Table 3.

TABLE 3

| Example | Composition of catalyst precursor (molar ratio) | Reaction temperature (°C) | Conversion of carbon monoxide*1 (%) | Selectivity of alcohol*2 (%) | Composition of mixed alcohol (% by weight) | | | |
|---|---|---|---|---|---|---|---|---|
| | | | | | Methanol | Ethanol | Propanol | Butanol and higher alcohols than butanol |
| 17 | $Zn \cdot Co_{0.5} \cdot Mg \cdot Na_{0.3}$ | 288 | 20 | 47 | 54 | 28 | 10 | 8 |
| 18 | $Zn \cdot Co_{0.5} \cdot Al \cdot Na_{0.3}$ | 310 | 20 | 56 | 50 | 31 | 11 | 8 |
| 19 | $Zn \cdot Co_{0.5} \cdot Al \cdot Mg_{0.3}$ | 334 | 19 | 53 | 70 | 22 | 5 | 3 |
| 20 | $Zn \cdot Co_{0.5} \cdot Ga \cdot Na_{0.3}$ | 312 | 21 | 54 | 52 | 30 | 11 | 7 |
| 21 | $Zn \cdot Co_{0.5} \cdot Si \cdot Na_{0.3}$ | 320 | 22 | 50 | 51 | 29 | 12 | 8 |
| 22 | $Zn \cdot Co_{0.5} \cdot Zr \cdot K_{0.3}$ | 288 | 20 | 42 | 45 | 31 | 11 | 3 |
| 23 | $Zn \cdot Co_{0.5} \cdot Cr \cdot Na_{0.3}$ | 335 | 20 | 62 | 72 | 20 | 6 | 2 |
| 24 | $Zn \cdot Co_{0.5} \cdot La \cdot Mg_{0.3}$ | 306 | 20 | 41 | 45 | 28 | 18 | 9 |
| 25 | $Zn \cdot Ni_{0.5} \cdot Al \cdot K_{0.3}$ | 388 | 18 | 58 | 46 | 19 | 28 | 7 |
| 26 | $Zn \cdot Ni_{0.5} \cdot Zr \cdot K_{0.3}$ | 367 | 19 | 64 | 50 | 25 | 20 | 5 |
| 27 | $Zn \cdot Ni_{0.5} \cdot Ti \cdot Na_{0.4}$ | 360 | 21 | 65 | 75 | 14 | 8 | 3 |
| 28 | $Zn \cdot Fe_{0.5} \cdot Al \cdot Na_{0.3}$ | 280 | 22 | 49 | 49 | 31 | 12 | 8 |
| 29 | $Zn \cdot Fe_{0.5} \cdot Zr \cdot Na_{0.3}$ | 282 | 21 | 48 | 45 | 34 | 13 | 8 |

Note:
*1, *2Same as in Table 1.

0 110 357

Example 30

One and half liters of an aqueous solution (Aqueous Solution I) containing 24.2 grams of copper nitrate (3 hydrate), 29.1 grams of nickel nitrate (6 hydrate), and 80 grams of titanium sulfate solution (Ti(SO$_4$)$_2$ content: 30% by weight) was prepared and heated to 60°C. Separately 1.5 liters of an aqueous solution (Aqueous Solution II) containing 66.3 grams of sodium carbonate (anhydrous) was prepared and heated to 90°C. These aqueous solutions I and II were mixed rapidly and maintained at 85°C for about 2 hours with stirring vigorously to be precipitated. The pH of the above described solution was 9.2. Then the solution containing the precipitate was filtered, and the thus obtained precipitate was washed with water in 200 fold amount of the precipitate.

The precipitate was dried at 120°C for about 10 hours and then was calcined at 450°C for 2 hours. The thus calcined product was cooled to a room temperature, and then the product was impregnated with 19.2 milliliters of an aqueous solution (Aqueous Solution III) of sodium carbonate (concentration: 1.0 mole per liter) in the water bath heated at 90°C and evaporated to dryness. The thus-obtained product was dried at 120°C for about 5 hours. Then graphite of 2% by weight based on the product was added thereto, and the resulting mixture was pelletized (catalyst precursor). Sulfur content in the thus prepared catalyst precursor is 0.2% by weight.

The catalyst precursor was reduced in the same manner as in Example 1 to form a catalyst. Using the thus-prepared catalyst, the production of mixed alcohol from synthesis gas was performed in the same manner as in Example 1 except that the reaction pressure was gradually increased to 61 kilograms per square centimeter (by gauge).

The results are shown in Table 4.

Example 31

A catalyst precursor was prepared in the same manner as in Example 30 except that 1.5 liters of an aqueous solution containing 199.0 grams of sodium carbonate (anhydrous) was used as Aqueous Solution II and the pH of the above described solution was 9.9. Sulfur content in the thus-prepared catalyst precursor was 0.1% by weight.

The catalyst precursor was reduced in the same manner as in Example 1 to form a catalyst. Using the thus-prepared catalyst, the production of mixed alcohol from synthesis gas was performed in the same manner as in Example 1 except that the reaction pressure was gradually increased to 61 kilograms per square centimeter (by gauge).

The results are shown in Table 4.

Example 32

One and half liters of an aqueous solution (Aqueous Solution I) containing 24.2 grams of copper nitrate (3 hydrate), 29.1 grams of nickel nitrate (6 hydrate), and 80 grams of titanium sulfate solution (Ti(SO$_4$)$_2$ content: 30% by weight) was prepared and heated to 60°C. Separately 1.5 liters of an aqueous solution (Aqueous Solution II) containing 66.3 grams of sodium carbonate (anhydrous) was prepared and heated to 90°C. These aqueous solutions I and II were mixed rapidly and maintained at 85°C for about 2 hours with stirring vigorously to be precipitated. The pH of the above described solution was 9.3. Then the solution containing the precipitate was filtered, and thus obtained precipitate was washed with water in 200 fold amount of the precipitate.

The precipitate was sufficiently suspended in 2 liters of an aqueous solution (80°C) of sodium chloride (concentration: 0.5 mole per liter), and was separated by filtration. Then the precipitate was washed again with water in 200 fold amount of the precipitate.

The precipitate was dried at 120°C for about 10 hours and then calcined at 450°C for 2 hours. The thus-calcined product was cooled to a room temperature, and then the product was impregnated with 19.2 milliliters of an aqueous solution (Aqueous Solution III) of sodium carbonate (concentration: 1.0 mole per liter) in the water bath heated at 90°C and evaporated to dryness. The thus-obtained product was dried at 120°C for about 5 hours. Then graphite of 2% by weight based on the product was added thereto, and the resulting mixture was pelletized (catalyst precursor). Sulfur content in the thus-prepared catalyst precursor is 0.2% by weight.

The catalyst precursor was reduced in the same manner as in Example 1 to form a catalyst. Using the thus-prepared catalyst, the production of mixed alcohol from synthesis gas was performed in the same manner as in Example 1 except that the reaction pressure was gradually increased to 61 kilograms per square centimeter (by gauge).

The results are shown in Table 4.

Example 33

A catalyst precursor was prepared in the same manner as in Example 32 except that 1.5 liters of an aqueous solution containing 153.0 grams of sodium carbonate (anhydrous) was used as Aqueous Solution II and the pH of the above solution was 8.0. Sulfur content in the thus-prepared catalyst precursor was 0.5% by weight.

The catalyst precursor was reduced in the same manner as in Example 1 to form a catalyst. Using the thus-prepared catalyst, the production of mixed alcohol from synthesis gas was performed in the same manner as in Example 1 except that the reaction pressure was gradually increased to 61 kilograms per square centimeter (by gauge).

The results are shown in Table 4.

## TABLE 4

Composition of oxygen-containing compounds formed (% by weight)

| Example | Reaction temperature (°C) | Conversion of carbon monoxide[1] (%) | Selectivity of oxygen-containing compounds[2] (%) | Methanol | Ethanol | Propanol | Butanol and higher alcohols than butanol | Dimethyl ether | Acet-aldehyde | Acetone | Methyl ethyl ketone |
|---|---|---|---|---|---|---|---|---|---|---|---|
| 30 | 297 | 19 | 58 | 56 | 31 | 4 | 4 | <1 | 2 | 2 | <1 |
| 31 | 297 | 19 | 65 | 57 | 23 | 11 | 6 | <1 | 1 | 0 | <1 |
| 32 | 307 | 20 | 54 | 55 | 33 | 5 | 4 | 0 | 1 | 2 | <1 |
| 33 | 307 | 19 | 53 | 59 | 24 | 12 | 3 | 0 | 0 | 2 | 0 |

Note:

[1]Conversion of Carbon Monoxide=
$$\frac{\text{Amount of Carbon Monoxide Converted into Alcohol (moles)} - \text{Amount of Carbon Dioxide Formed (moles)}}{\text{Amount of Carbon Monoxide Introduced (moles)}} \times 100$$

[2]Selectivity of Oxygen-containing Compounds=
$$\frac{\text{Amount of Carbon Monoxide Converted into Oxygen-containing Compounds (moles)}}{\text{Amount of Carbon Monoxide Converted (moles)} - \text{Amount of Carbon Dioxide Formed (moles)}} \times 100$$

**Claims**

1. A process for producing a mixed alcohol comprising methanol and higher alcohol than methanol by contacting a synthesis gas with a catalyst, wherein the catalyst is a solid substance prepared by: calcining a mixture of $(A_1)$ a copper compound, $(B_1)$ a nickel compound, and (C) a compound of at least one metal selected from the metals belonging to Groups II, III and IV and the fourth period of Groups V, VI and VII of the Periodic Table; impregnating the above-calcined product with (D) an alkali metal compound and/or an alkaline earth metal compound; calcining the resulting mixture; and reducing the thus-calcined product.

2. The process for the production of mixed alcohols as claimed in Claim 1, wherein the Compound (C) is a compound of at least one metal selected from the groups consisting of magnesium, zinc, aluminum, gallium, lanthanum, silicon, titanium, zirconium, chromium and manganese.

3. The process for the production of mixed alcohols as claimed in Claim 1, wherein the Compound (D) is sodium compound or magnesium compound.

4. A process for producing a mixed alcohol comprising methanol and higher alcohols than methanol by contacting a synthesis gas with a catalyst, wherein the catalyst is a solid substance prepared by: calcining a mixture of $(A_2)$ a zinc compound, $(B_2)$ a compound of at least one metal selected from iron, cobalt, and nickel, and (C) a compound of at least one metal selected from the metals belonging to Groups II, III and IV and the fourth period of Groups V, VI and VII of the Periodic Table; impregnating the above-calcined product with (D) an alkali metal compound and/or an alkaline earth metal compound; calcining the resulting mixture; and reducing the thus-calcined product.

5. The process for the production of mixed alcohols as claimed in Claim 4, wherein the Compound (C) is a compound of at least one metal selected from the groups consisting of magnesium, aluminum, gallium, lanthanum, silicon, titanium, zirconium, chromium and manganese.

6. The process for the production of mixed alcohols as claimed in Claim 4, wherein the Compound (D) is a compound of at least one metal selected from the groups consisting of sodium, magnesium and potassium.

**Patentansprüche**

1. Verfahren zur Herstellung eines gemischten Alkohols, der Methanol und höhere Alkohole als Methanol umfaßt, durch Kontakt eines Synthesegases mit einem Katalysator, bei dem der Katalysator eine feste Substanz ist, hergestellt durch: Kalzinieren eines Gemisches von $(A_1)$ einer Kupferverbindung, $(B_1)$ einer Nickelverbindung und (C) einer Verbindung von mindestens einem Metall, ausgewählt aus den Metallen, die den Gruppen II, III und IV und der vierten Periode der Gruppen V, VI und VII der Periodensystems angehören; Imprägnieren des vorstehenden kalzinierten Produkts mit (D) einer Alkalimetallverbindung und/oder einer Erdalkalimetallverbindung; Kalzinieren des resultierenden Gemischs; und Reduzieren des so kalzinierten Produkts.

2. Verfahren zur Herstellung gemischter Alkohole, wie in Anspruch 1 beansprucht, worin die Verbindung (C) eine Verbindung von mindestens einem Metall ist, ausgewählt aus den Gruppen bestehend aus Magnesium, Zink, Aluminium, Gallium, Lanthan, Silizium, Titan, Zirkonium, Chrom und Mangan.

3. Verfahren zur Herstellung gemischter Alkohole, wie in Anspruch 1 beansprucht, bei dem die Verbindung (D) eine Natriumverbindung oder Magnesiumverbindung ist.

4. Verfahren zur Herstellung eines gemischten Alkohols, der Methanol und höhere Alkohole als Methanol umfaßt, durch Kontakt eines Synthesegases mit einem Katalysator, bei dem der Katalysator eine feste Substanz ist, hergestellt durch: Kalzinieren eines Gemischs von $(A_2)$ einer Zinkverbindung, $(B_2)$ einer Verbindung von mindestens einem Metall, ausgewählt aus Eisen, Kobalt und Nickel und (C) einer Verbindung mindestens eines Metalls, ausgewählt aus den Metallen, die den Gruppen II, III, und IV und der vierten Periode der Gruppen V, VI und VII des Periodensystems angehören; Imprägnieren des vorstehend kalzinierten Produkts mit (D) einer Alkalimetallverbindung und/oder einer Erdalkalimetallverbindung; Kalzinieren des resultierenden Gemischs; und Reduzieren des so kalzinierten Produkts.

5. Verfahren zur Herstellung gemischter Alkohole, wie in Anspruch 4 beansprucht, wobei die Verbindung (C) eine Verbindung mindestens eines Metalls ist, ausgewählt aus den Gruppen bestehend aus Magnesium, Aluminium, Gallium, Lanthan, Silizium, Titan, Zirkonium, Chrom und Mangan.

6. Verfahren zur Herstellung gemischter Alkohole, wie in Anspruch 4 beansprucht, wobei die Verbindung (D) eine Verbindung mindestens eines Metalls ist, ausgewählt aus den Gruppen bestehend aus Natrium, Magnesium und Kalium.

**Revendications**

1. Procédé de préparation d'un mélange d'alcools comprenant du méthanol et des alcools plus lourds que le méthanol en mettant en contact un gaz de synthèse avec un catalyseur, dans lequel le catalyseur est un matériau solide préparé:

en calcinant un mélange d'un composé ($A_1$) à base de ouivre d'un composé ($B_1$) à base de nickel et d'un composé (C) comprenant au moins un métal choisi parmi les métaux appartenant aux groupes II, III et IV et à la quatrième période des groupes V, VI et VII du tableau périodique;

en imprégnant le produit calcine ci-dessus avec un composé (D) à base d'un métal alcalin et/ou un composé à base d'un métal alcalino-terreux;

en calcinant le mélange résultant; et

en réduisant le produit ainsi calciné.

2. Procédé de préparation d'un mélange d'alcools suivant la revendication 1, dans lequel le composé (C) est un composé comprenant au moins un métal choisi parmi le magnésium, le zinc, l'aluminium, le gallium, le lanthane, le silicium, le titane, le zirconium, le chrome et le manganèse.

3. Procédé de préparation d'un mélange d'alcools suivant la revendication 1, dans lequel le composé (D) est un composé à base de sodium, ou un composé à base de magnésium.

4. Procédé de préparation d'un mélange d'alcools comprenant du méthanol et des alcools plus lourds que le méthanol en mettant en contact un gaz de synthèse avec un catalyseur, dans lequel le catalyseur est un matériau solide préparé:

en calcinant un mélange d'un composé ($A_2$) à base de zinc, d'un composé ($B_2$) comprenant au moins un métal choisi parmi le fer, le cobalt et le nickel et un composé (C) comprenant au moins un métal choisi parmi les métaux appartenant aux groupes II, III, et IV et à quatrième période des groupes V, VI et VII du tableau périodique;

en imprégnant le produit calciné ci-dessus avec un composé (D) à base d'un métal alcalin et/ou un composé à base d'un métal alcalino-terreux;

en calcinant le mélange résultant; et

en réduisant le produit ainsi calciné.

5. Procédé de préparation d'un mélange d'alcools suivant la revendication 4, dans lequel le composé (C) est un composé contenant au moins un métal choisi parmi les groupes comprenant le magnésium, l'aluminium, le gallium, le lanthane, le silicium, le titane, le zirconium, le chrome et le manganèse.

6. Procédé de préparation d'u mélange d'alcools suivant la revendication 4, dans lequel le composé (D) est un composé contenant au moins un métal choisi parmi les groupes comprenant le sodium, le magnésium et le potassium.